# EUROPEAN PATENT SPECIFICATION

(11) **EP 1 969 307 B1**
(45) Date of publication and mention of the grant of the patent: **04.08.2010**
(21) Application number: 06805600.1
(22) Date of filing: 28.11.2006
(51) Int. Cl.: G01B 11/25, A61B 1/227, A61B 5/107

(54) **CODED STRUCTURED LIGHT**
CODIERTES STRUKTURIERTES LICHT
LUMIERE STRUCTUREE CODEE

(30) Priority: 28.11.2005 DK 200501669
(43) Date of publication of application: 17.09.2008
(73) Proprietor: 3Shape A/S, 1060 Copenhagen (DK)
(72) Inventor: VINTHER, Michael, DK-2300 Copenhagen S (DK); CLAUSEN, Tais, DK-2100 Copenhagen Oe (DK); FISKER, Rune, DK-2830 Virum (DK); DEICHMANN, Nikolaj, DK-2100 Copenhagen Ø (DK)
(74) Representative: Høiberg A/S
(86) International application number: PCT/DK2006/000664
(87) International publication number: WO 2007/059780

(56) References cited:
- JP-A- 2 110 305
- US-A- 5 680 216
- KONINCKX T P ET AL: "Real-time range scanning of deformable surfaces by adaptively coded structured light" 3-D DIGITAL IMAGING AND MODELING, 2003. 3DIM 2003. PROCEEDINGS. FOURTH INTERNATIONAL CONFERENCE ON 6-10 OCT. 2003, PISCATAWAY, NJ, USA,IEEE, 6 October 2003 (2003-10-06), pages 293-300, XP010662707 ISBN: 0-7695-1991-1
- SALVI J ET AL: "Pattern codification strategies in structured light systems" PATTERN RECOGNITION, ELSEVIER, KIDLINGTON, GB, vol. 37, no. 4, April 2004 (2004-04), pages 827-849, XP004491495 ISSN: 0031-3203 cited in the application
- MINORU MARUYAMA ET AL: "RANGE SENSING BY PROJECTING MULTIPLE SLITS WITH RANDOM CUTS" IEEE TRANSACTIONS ON PATTERN ANALYSIS AND MACHINE INTELLIGENCE, IEEE SERVICE CENTER, LOS ALAMITOS, CA, US, vol. 15, no. 6, 1 June 1993 (1993-06-01), pages 647-651, XP000369964 ISSN: 0162-8828

## Description

The present invention relates to a system and a method for creating a three-dimensional model of a surface using coded structured light.

### Background of invention

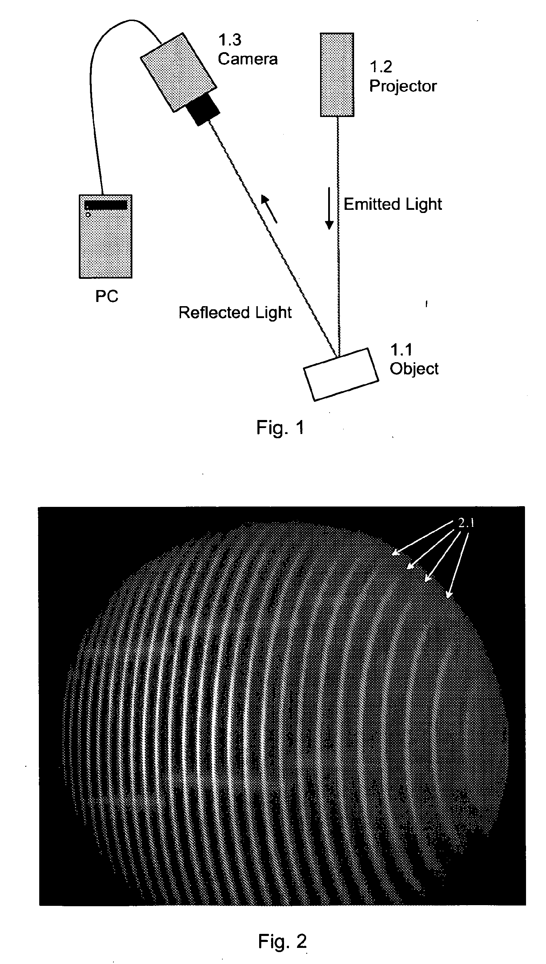

A method for producing a digital three-dimensional model of a physical object [1.1] is to project a known light pattern [1.2] onto the surface of the object, record the projected pattern with a camera [1.3] from a different angle (Figure 1) and then compute the shape of the surface from the recorded deformation of the pattern. When the relative positions and the internal parameters of the projector and the camera are known then the three-dimensional shape of the illuminated part of the object can be computed using triangulation. This is known as structured light scanning and described in the prior art.

The identification of features in the pattern presents a problem that has been solved in a number of different ways in existing systems. Salvi et al. (2004) gives an extensive overview of existing pattern coding strategies. The primary categories are:
- Projecting only a single line. If only a single line is projected there is no risk of erroneous identification (assuming that there is no external illumination of the object), but only a single thin stripe of the object's surface will be covered so a large number of scans from different angles will be necessary to cover the surface. This takes time and requires controlled movement of the object or projector and camera which adds complexity to the scanner system.
- Projecting lines or dots coded with different colors or grayscales. Individual lines or dots can be identified if they have different colors/grayscales provided that the object is of (almost) uniform color or that an image of the object can be recorded in uniform illumination. Acquiring images of the object both in uniform illumination and with the projected pattern requires that the system is stationary between the two recordings and this is thus not suitable for hand-held scanners or moving objects. Furthermore using color introduces potential inaccuracies (reduced resolution) because light of different colors has different angles of refraction in the camera and projector lenses, and because of the technology used in typical camera chips. An example of a color coded light system can be found in US Patent No 6,147,760. Pages et al. (2004) and JP 02110305 A describe systems that applies colored lines.
- Time-varying patterns. Projecting several different patterns where e.g. different lines are visible can give certain line identification, but again this requires acquiring several images with stationary object, camera and projector. It also requires a projector capable of changing the pattern and such a projector will be more expensive than one with a fixed image. An example of a system applying a time-varying coding is described in US Patent No 4,653,104.

US 5,680,216 provides an example of raster-stereographic (20) measurements by means of projecting a plurality of modulated raster lines onto a surface.

Scanning in a small cavity as e.g. the mouth or the ear canal limits the possible size of a scanner, and furthermore a handheld device will often be the most user-friendly and cost-efficient solution for such an application. If the scanner is handheld one cannot expect to have a stationary scene over time, even if the user is instructed to hold the device steady. This means that time-varying patterns will be problematic and that the movement between the consecutively acquired images may be unknown, so it is desirable to have as much information as possible in a single image.

### Summary of invention

The present invention provides a solution to the above-mentioned problems in that the present invention provides a system and a method that are usable in relation to a dynamic scene since the present invention offers computing from a single-frame (ie. one-shot) image in order to provide a three-dimensional model.

Accordingly, in one aspect the present invention relates to a system for creating a three-dimensional model of a surface comprising
(a) a light source that projects a pattern of continuous line segments onto the surface, wherein each line segment is coded with a unique pattern of varying line width or intensity along the line segment,
(b) a detector that records an image of the surface with said projected pattern, and
(c) a computer for transforming said image to a three-dimensional model of the surface utilizing said projected pattern.

In a further aspect the invention relates to a method for creating a three-dimensional model of a surface comprising the steps of
(a) from a light source projecting a pattern of continuous line segments onto the surface, wherein each line segment is coded with a unique pattern of varying line width or intensity along the line segment,
(b) recording an image of the surface with said projected pattern, and
(c) transforming said image to a three-dimensional model of the surface utilizing said projected pattern.

### Description of Drawings

Figure 1: Structured light scanner with camera and projector.
Figure 2: Structured light pattern projected onto a simple surface.
Figure 3: Structured light pattern projected onto a complex surface.
Figure 4: Binary coding along lines.
Figure 5: Frequency coding with eight different frequencies and two sequences with different phase.
Figure 6: Vertical height and position of bits is preserved independently of the object's shape.
Figure 7: An ear with projected bit coded pattern.
Figure 8: Slide with coded line pattern.
Figure 9: Interpolating the surface between lines using triangles.

### Definitions

Along the line: means in the direction of the line.

Continuous line segment: means a line segment of continuous points or pixels, having no visible gaps on the image.

Three-dimensional model: A set of data representing the spatial distribution of the surface of the object being modeled within the accuracy of the data collection process.

Unique pattern: A predetermined recognizable modulation of a line segment identifying said line segment either relative to any other line segment projected by the light source or relative to proximal line segments. A unique pattern may be repeated in line segments belonging to the same line. In another definition, a unique pattern is a predetermined recognizable modulation of a line segment making said line segment distinguishable from any other line segment projected by the light source or distinguishable from close line segments. Here close line segments are defined as line segments wherein a line segment viewed by the detector may be identified as originating from the correct original line segment projected by the light source or identified as a close line segment. Said identification may also be more or less ambiguous between the correct and any close line segments. A unique pattern may be repeated in line segments belonging to the same line.

Frequency and phase: A sinusoidal modulation of a line segment where said modulation is recognizable through the frequency and/or phase of said modulation. The phase of said modulation is often measured relative to a reference, such as an identifiable point, line or other pattern.

### Detailed description of the invention

The objective of the invention is a new improved coding method that solves the problem of identifying the projected lines in a structured light scanner, whereby the coding method may be used in a simple and cheap embodiment of small physical size. The projected light pattern consists of a pattern of continuous line segments. Each line segment is provided with a unique coding. In one embodiment the continuous line segments are arranged in lines, whereby said lines are consisting of the continuous line segments. The line segments may be arranged in a line with a gap between two continuous line segments, or the line segments may be arranged in a continuity in the line. Lines consisting of continuous line segments are arranged having a predetermined distance from one line to the next, such as parallel lines, when projected onto the surface. In one embodiment the continuous line segment are straight continuous line segments.

The unique coding along the continuous line segments may be carried out in any suitable manner allowing identification of each continuous line segment in the image. In one embodiment the same unique pattern is coded along all continuous line segments in a line, it is however also possible to vary the unique coding pattern from continuous line segment to continuous line segment in a line, as long as it is possible to identify one line from neighbouring lines.

The unique coding pattern may be any suitable pattern that may be applied along the continuous line segments. Accordingly, the unique pattern may consist of a periodically change in the width of the continuous line segment, such as the examples shown in the Figures of this application.

In combination therewith the unique coding pattern may consist of a periodically change in colour in the continuous line segment. For example a line segment may consist of alternating red and green parts along the line segment.

Furthermore, the unique coding pattern may consist of a periodically change in greyscale in the continuous line segment either alone or in combination with any of the above mentioned coding patterns.

The pattern may be unique for each line or continuous line segment in the image. However, in practice it is only necessary that the uniqueness of the pattern is sufficient to distinguish it from immediate neighbour lines. Therefore, in one embodiment the unique pattern is repeated for every n lines in the pattern, and n is an integer of at least 2, such as at least 3, such as at least 4, such as at least 5, such as at least 10, such as at least 25.

As described below in greater detail, the continuous line segments may be coded using a binary or n-ary sequence or by changing frequency and/or phase.

The line segments as defined herein are continuous, wherein the term continuous is used in its conventional meaning, i.e. that there are no gaps in the continuous line segment. The provision of continuous line segments provides for a more effective transformation of the image into a three-dimensional model, since even a short part of a continuous line segment may be identified, because no gap disturbs the identification process.

In one embodiment it is preferred that the length of each continuous line segment in the image is at least two times the smallest width of the continuous line segment, such as at least three times the smallest width of the continuous line segment, such as at least four times the smallest width of the continuous line segment, such as at least five times the smallest width of the continuous line segment, such as at least ten times the smallest width of the continuous line segment, such as at least 25 times the smallest width of the continuous line segment, such as at least 50 times the smallest width of the continuous line segment.

In a further embodiment the light source further projects lines having a predetermined angle in relation to the continuous line segments onto the surface, such as lines being perpendicular to the continuous line segments.

It is preferred that the coded lines segments in the image are perpendicular to the axis between the focal line of the detector and the light source, such as described in further details below.

The light source used according to the present invention may be any suitable light source. Accordingly, any structured light may be used, such as the light source in a conventional projector, or a laser light, or a blitz light. The light source may emit visible light, near-visible or invisible light as is suitable for the image and the surface. In particular for creating a three-dimensional model of a surface of a human being or an animal it may be preferred to use invisible light.

The detector according to the present invention may be any suitable detector, such as a digital camera. The system may include two or more detectors if suitable.

As an example the present invention may be used in a system as described in any of the patent applications PCT/DK01/00564 and PCT/DK2005/000507.

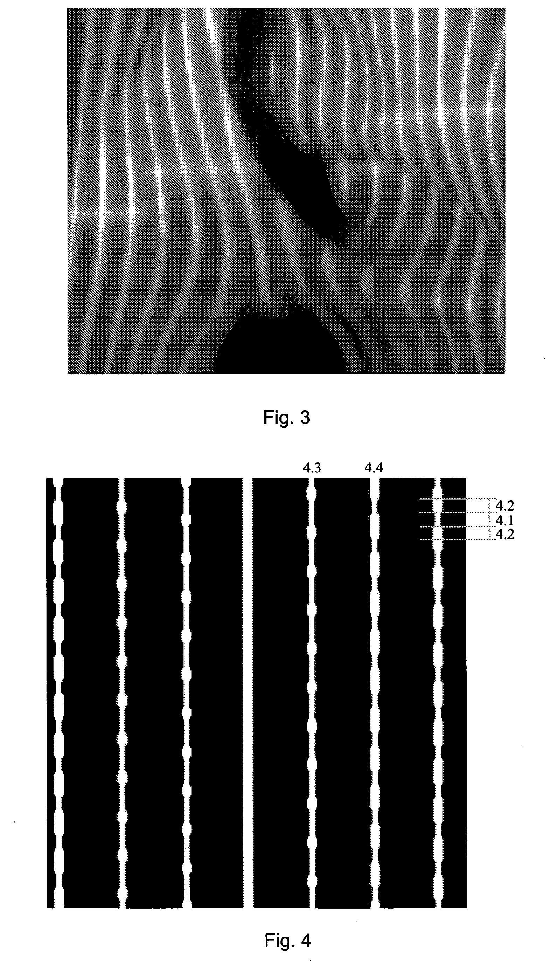

By projecting a pattern of continuous line segments on the surface it is possible to create a three-dimensional model from an image of the surface. To reconstruct the surface from the recorded image, it must be possible to identify the projected features in the recording, i.e. the individual lines. Figure 2 shows a pattern of lines [2.1] projected onto a ball. In Figure 3 the same pattern is projected onto a more complex surface where determining which segments belong to which line is far more complicated to do in an automated procedure.

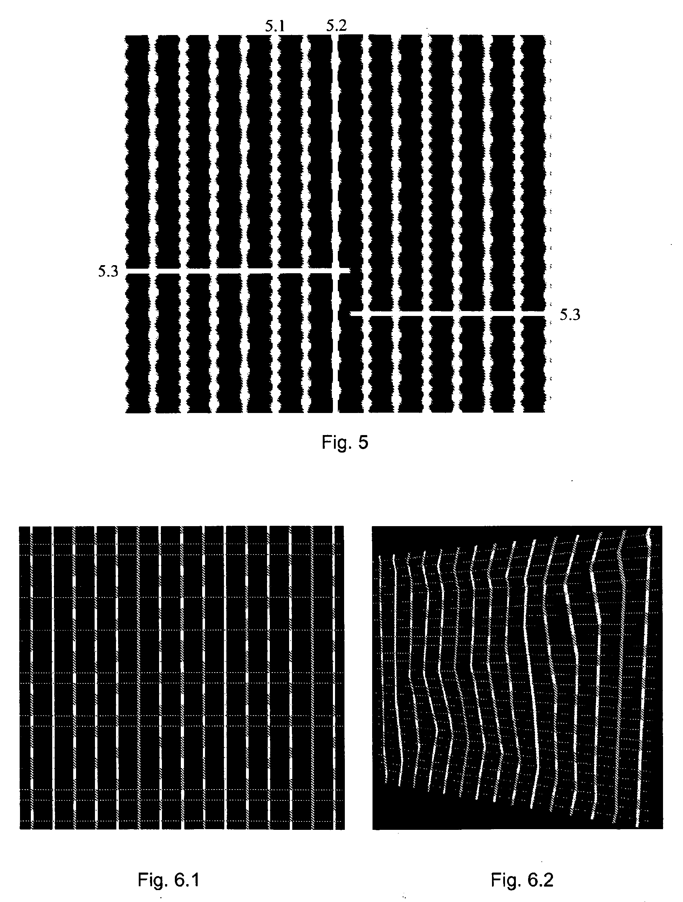

To be able to distinguish the projected lines in a recorded image the invention proposes using a coding *along* the lines as varying line width or intensity. This could e.g. be a binary coding as shown in Figure 4 or a frequency and/ phase coding as shown in Figure 5.

With a binary coding one could define the length of a bit [4.1]/[4.2] to be e.g. 1/100 of the total height of the projected image and a thin line [4.1] as 0 and a wide line [4.2] as 1. This would give the line [4.3] the code 010010010... (top-down) and the line [4.4] the code 110110110.... With just a short segment of a line, in this case at least corresponding to the length of 3 bits, one is able to identify the segment.

In another embodiment of the invention the line width could change as a sinusoidal function of the distance from the top with different frequency and phase for each line. In the example in Figure 5 one can see that the line [5.1] has a higher frequency than the line [5.2]. A Fourier transform of a band of pixel values along a line segment in the recorded image will give the frequency that identifies the line. The length of a line segment should preferably be at least as long as the cycle of the sinusoidal for certain identification.

It is important to realize that the vertical position of the bits (in case of bit coding) and the line frequency (in case of frequency coding) in the recorded image is not affected by the shape of the object but only by the relative position and orientation of the projector and camera. This is true if the coded lines in the source image in [1.2] are perpendicular to the axis between the focal line of the camera and the projector. The shape of the object only shifts the lines perpendicular so the coding is preserved in the recorded image.

As illustrated in Figure 6 a rotation of the projector relative to the camera gives a linear transformation of vertical features on the lines. If [6.1] is the source image then [6.2] could be the image recorded when projecting [6.1] onto an irregular object. The illustration demonstrates that the lines are shifted horizontally depending on the surface but the vertical positions of the bits are only linearly transformed because of the projector/camera rotation. The inverse linear transformation can be applied to the recorded image for simpler line identification.

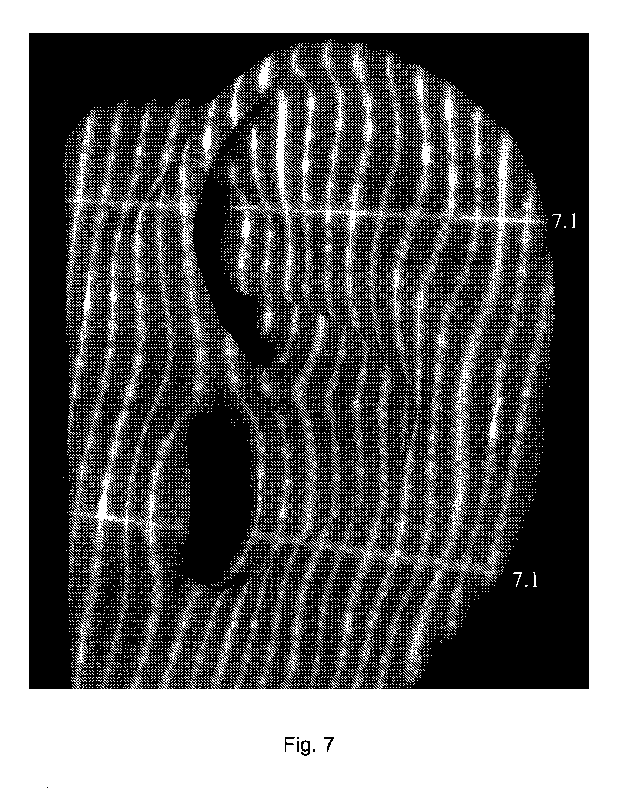

Another example of this property is shown in Figure 7 where the direction of the horizontal lines [7.1] are clearly unaffected by the varying surface of an ear.

Determining the linear transformation of the coding and other system parameters needed for obtaining absolute object measurements can be done by recoding a number of calibration images with an object of known dimensions. To support the calibration process a number of horizontal lines [5.3] [7.1] can be inserted in the source image.

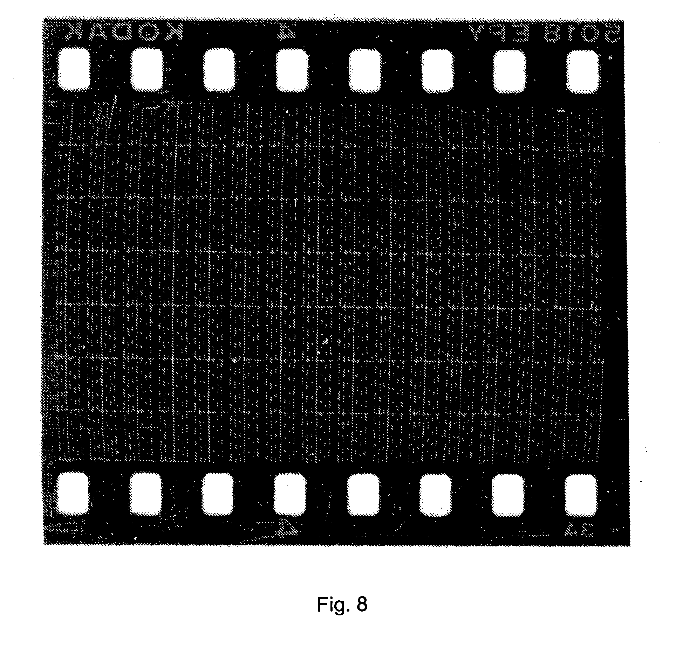

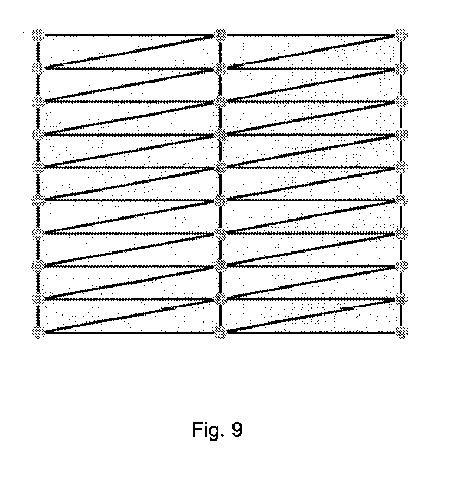

The scanner hardware of the system and the method may consist of a projector and a camera. The projector could be a simple slide projector where the slide contains the coded lines (see Figure 8), it could be a LCD/DMD projector or the pattern could be generated by one or more lasers. A TV-camera or digital camera (typically CCD or CMOS based) connected to a computer supplies the images. A number of algorithms for detecting lines in digital images are known in the prior art. Assuming that there is no other light on the object than that from the projector a simple threshold approach can be used, where all pixel values above a threshold value are considered as being part of a line. If the lines are wider than one pixel in the recorded image the center must be determined by e.g. finding the brightest pixel or as the maximum of a polynomial fitted through the pixel values across the line. Once the lines are found they must be identified based on the coding as described above, and at last the three dimensional position of each pixel along each line can be computed using triangulation. Algorithms for connecting the points in space to a continuous surface are also described in the prior art. One way to do this is to connect neighbouring points with triangles as shown in Figure 9.

The invention may be applied in any scanning of surfaces for producing three-dimensional models, in particular in relation to hand-held scanners and/or dynamic scenes. Therefore, the invention has many possible applications. One could be hand-held small cavity scanners for use in the hearing aid or dental industry. More and more hearing aids are custom made from a 3D model of the patient's ear, and methods for acquiring this 3D model as fast and painless as possible for the patients are desired. Likewise dental restorations and orthodontics are frequently based on a digital 3D model of the patient's mouth. Thus, surface may be the surface of the auditory canal of a person or a surface of a three-dimensional model of the auditory canal. In another embodiment the surface is a teeth or tooth surface or a surface of a three-dimensional model of teeth or a tooth.

Other applications are scanning of objects for quality control in mass production, quality control, reverse engineering, virtual reality and computer game model making, mould making or scanning of hand made clay models for design.

### References

US Patent No 4,653,104 (binary coding over time).
US Patent No 6,147,760 (rainbow colored light).
J. Pagès and J. Salvi. A new optimised De Bruijn coding strategy for structured light patterns. 17th International Conference on Pattern Recognition, ICPR 2004, Cambridge, UK, Volume: 4, Aug. 23-26, 2004, Pages: 284 - 287.
J. Salvi, J. Pagès, J. Batlle. Pattern Codification Strategies in Structured Light Systems. Pattern Recognition 37(4), pp 827-849, April 2004.

## Claims

1. A system for creating a three-dimensional model of a surface comprising
(a) a light source (1.2) that projects a pattern of continuous line segments onto the surface, wherein a continuous line segment is a line segment of continuous points or pixels having no visible gaps and wherein each line segment is coded with a unique pattern of varying line width or intensity along the line segment,
(b) a detector (1.3) that records an image of the surface with said projected pattern, and
(c) a computer (PC) for transforming said image to a three-dimensional model of the surface utilizing said projected pattern.

2. The system according to claim 1, wherein the light source projects a pattern of lines onto the surface, each line consisting of a plurality of said continuous line segments.

3. The system according to claim 2, wherein each line consists of a continuity of said continuous line segments.

4. The system according to any of the preceding claims, wherein the same unique pattern is coded along all continuous line segments in a line.

5. The system according to any of the preceding claims, wherein the unique pattern consists of a periodically change in the width of the line segment.

6. The system according to claim 5, wherein in combination with the change in width, the unique pattern consists of a periodically change in colour in the line segment.

7. The system according to any of the preceding claims, wherein the unique pattern consists of a periodically change in greyscale in the line segment.

8. The system according to any of the preceding claims, wherein the unique pattern is repeated for every n lines in the pattern, and n is an integer of at least 2.

9. The system according to any of the preceding claims, wherein the continuous line segments are coded using a binary sequence.

10. The system according to any of the preceding claims, wherein the continuous line segments are coded by changing frequency and/or phase of the pattern.

11. The system according to any of the preceding claims, wherein the length of each continuous line segment is at least two times the smallest width of the continuous line segment.

12. The system according to any of the preceding claims, wherein the light source emits visible light.

13. The system according to any of the preceding claims 1-11, wherein the light source emits invisible light.

14. The system according to any of the preceding claims, wherein the detector is a camera.

15. The system according to claim 1, wherein the light source further projects lines having a predetermined angle in relation to the continuous line segments onto the surface.

16. A method for creating a three-dimensional model of a surface comprising the steps of
(a) from a light source projecting a pattern of continuous line segments onto the surface, wherein a continuous line segment is a line segment of continuous points or pixels having no visible gaps and wherein each line segment is coded with a unique pattern of varying line width or intensity along the line segment,
(b) recording an image of the surface with said projected pattern, and
(c) transforming said image to a three-dimensional model of the surface utilizing said projected pattern.

17. The method according to claim 16, carried out by the system of any of the claims 1-15.

18. The method according to claim 16 or 17, wherein the surface is the surface of the auditory canal of a person or a surface of a three-dimensional model of the auditory canal.

19. The method according to claim 16 or 17, wherein the surface is a teeth or tooth surface or a surface of a three-dimensional model of teeth or a tooth.

## Patentansprüche

1. System zum Erzeugen eines dreidimensionalen Modells einer Oberfläche, umfassend
(a) eine Lichtquelle (1.2), die ein Muster aus kontinuierlichen Liniensegmenten auf die Oberfläche projiziert, wobei ein kontinuierliches Liniensegment ein Liniensegment aus kontinuierlichen Punkten oder Pixeln ist, das keine sichtbaren Lücken aufweist, und wobei jedes Liniensegment mit einem individuellen Muster variierender Linienbreite oder Intensität entlang des Liniensegmentes kodiert ist,
(b) einen Detektor (1.3), der ein Bild der Oberfläche mit dem projizierten Muster aufzeichnet, und
(c) einen Computer (PC) zum Umformen des Bildes in ein dreidimensionales Modell der Oberfläche unter Verwendung des projizierten Musters.

2. System nach Anspruch 1, wobei die Lichtquelle ein Muster aus Linien auf die Oberfläche projiziert, wobei jede Linie aus einer Mehrzahl der kontinuierlichen Liniensegmente besteht.

3. System nach Anspruch 2, wobei jede Linie aus einer Kontinuität der kontinuierlichen Liniensegmente besteht.

4. System nach einem der vorrangehenden Ansprüche, wobei dasselbe individuelle Muster entlang aller kontinuierlichen Linienabschnitte in einer Linie kodiert ist.

5. System nach einem der vorrangehenden Ansprüche, wobei das individuelle Muster aus einer periodischen Änderung der Breite des Liniensegmentes besteht.

6. System nach Anspruch 5, wobei das individuelle Muster in Kombination mit der Änderung in der Breite aus einer periodischen Änderung der Farbe des Liniensegmentes besteht.

7. System nach einem der vorrangehenden Ansprüche, wobei das individuelle Muster aus einem periodischen Wechsel in der Grauskalierung des Liniensegmentes besteht.

8. System nach einem der vorrangehenden Ansprüche, wobei das individuelle Muster jede n-te Linie in dem Muster wiederholt wird, wobei n eine ganze Zahl mindestens gleich 2.

9. System nach einem der vorrangehenden Ansprüche, wobei die kontinuierlichen Liniensegmente unter Verwendung einer Binärsequenz kodiert sind.

10. System nach einem der vorrangehenden Ansprüche, wobei die kontinuierlichen Liniensegmente durch Änderung der Frequenz und/oder Phase des Musters kodiert sind.

11. System nach einem der vorrangehenden Ansprüche, wobei die Länge jedes kontinuierlichen Liniensegmentes wenigstens zweimal größer ist als die kleinste Breite des kontinuierlichen Liniensegmentes.

12. System nach einem der vorrangehenden Ansprüche, wobei die Lichtquelle sichtbares Licht emittiert.

13. System nach einem der vorrangehenden Ansprüche 1 bis 11, wobei die Lichtquelle unsichtbares Licht emittiert.

14. System nach einem der vorrangehenden Ansprüche, wobei der Detektor eine Kamera ist.

15. System nach Anspruch 1, wobei die Lichtquelle ferner Linien mit einem vorbestimmten Winkel relativ zu den kontinuierlichen Liniensegmenten auf die Oberfläche projiziert.

16. Verfahren zum Erzeugen eines dreidimensionalen Modells einer Oberfläche, umfassend die Schritte des
(a) Projizierens eines Musters von kontinuierlichen Liniensegmenten von einer Lichtquelle auf die Oberfläche, wobei ein kontinuierliches Liniensegment ein Liniensegment von kontinuierlichen Punkten oder Pixeln ohne sichtbare Lücken ist und wobei jedes Liniensegment mit einem individuellen Muster wechselnder Linienbreite oder Intensität entlang des Liniensegmentes kodiert ist,
(b) Aufzeichnens eines Bildes der Oberfläche mit dem projizierten Muster und
(c) Umformens des Bildes in ein dreidimensionales Modell der Oberfläche unter Verwendung des projizierten Musters.

17. Verfahren nach Anspruch 16, ausgeführt mit dem System nach einem der Ansprüche 1 bis 15.

18. Verfahren nach Anspruch 16 oder 17, wobei die Oberfläche die Oberfläche des Gehörgans einer Person oder eine Oberfläche eines dreidimensionalen Modells des Gehörgangs ist.

19. Verfahren nach Anspruch 16 oder 17, wobei die Oberfläche die Oberfläche eines Zahns oder von Zähnen oder eine Oberfläche eines dreidimensionalen Modells eines Zahns oder von Zähnen ist.

## Revendications

1. Système pour créer un modèle tridimensionnel d'une surface comprenant :
(a) une source de lumière (1, 2) qui projette un motif de segments de droite continus sur la surface, dans lequel un segment de droite continu est un segment de droite de points ou de pixels continus n'ayant aucun espace visible, et dans lequel chaque segment de droite est codé avec un motif unique de largeur ou d'intensité de droite variable le long du segment de droite,
(b) un détecteur (1, 3) qui enregistre une image de la surface avec ledit motif projeté, et
(c) un ordinateur (PC) pour transformer ladite image en un modèle tridimensionnel de la surface en utilisant ledit motif projeté.

2. Système selon la revendication 1, dans lequel la source de lumière projette un motif de droites sur la surface, chaque droite consistant en une pluralité desdits segments de droite continus.

3. Système selon la revendication 2, dans lequel chaque droite consiste en une continuité desdits segments de droite continus.

4. Système selon l'une quelconque des revendications précédentes, dans lequel le même motif unique est codé le long de tous les segments de droite continus sur une droite.

5. Système selon l'une quelconque des revendications précédentes, dans lequel le motif unique consiste en une variation périodique de la largeur du segment de droite.

6. Système selon la revendication 5, dans lequel, en combinaison avec la variation de largeur, le motif unique consiste en une variation périodique de couleur dans le segment de droite.

7. Système selon l'une quelconque des revendications précédentes, dans lequel le motif unique consiste en une variation périodique d'échelle de gris dans le segment de droite.

8. Système selon l'une quelconque des revendications précédentes, dans lequel le motif unique est répété toutes les n droites dans le motif, et n est un entier au moins égal à 2.

9. Système selon l'une quelconque des revendications précédentes, dans lequel les segments de droite continus sont codés en utilisant une séquence binaire.

10. Système selon l'une quelconque des revendications précédentes, dans lequel les segments de droite continus sont codés en modifiant la fréquence et/ou la phase du motif.

11. Système selon l'une quelconque des revendications précédentes, dans lequel la longueur de chaque segment de droite continu est égale à au moins deux fois la largeur la plus petite du segment de droite continu.

12. Système selon l'une quelconque des revendications précédentes, dans lequel la source de lumière émet une lumière visible.

13. Système selon l'une quelconque des revendications 1 à 11 précédentes, dans lequel la source de lumière émet une lumière invisible.

14. Système selon l'une quelconque des revendications précédentes, dans lequel le détecteur est une caméra.

15. Système selon la revendication 1, dans lequel la source de lumière projette en outre des droites ayant un angle prédéterminé en relation avec les segments de droite continus sur la surface.

16. Procédé pour créer un modèle tridimensionnel d'une surface comprenant les étapes consistant à :
(a) projeter à partir d'une source de lumière un motif de segments de droite continus sur la surface, dans lequel un segment de droite continu est un segment de droite de points ou de pixels continus n'ayant aucun espace visible, et dans lequel chaque segment de droite est codé avec un motif unique de largeur ou d'intensité de droite variable le long du segment de droite,
(b) enregistrer une image de la surface avec ledit motif projeté, et
(c) transformer ladite image en un modèle tridimensionnel de la surface en utilisant ledit motif projeté.

17. Procédé selon la revendication 16, exécuté par le système selon l'une quelconque des revendications 1 à 15.

18. Procédé selon la revendication 16 ou 17, dans lequel la surface est la surface du canal auditif d'une personne ou une surface d'un modèle tridimensionnel du canal auditif.

19. Procédé selon la revendication 16 ou 17, dans lequel la surface est une surface de dents ou d'une dent ou une surface d'un modèle tridimensionnel de dents ou d'une dent.
